# EUROPEAN PATENT SPECIFICATION

(11) **EP 1 961 389 B1**
(45) Date of publication and mention of the grant of the patent: **14.10.2009**
(21) Application number: 08250297.2
(22) Date of filing: 24.01.2008
(51) Int. Cl.: A61B 17/17

(54) **Apparatus for preparing a prosthetic stem cavity in a femur**
Vorrichtung zur Herstellung einer Knochenhohlraumprothese im Oberschenkelknochen
Appareil de préparation de cavité de tige de prothèse dans un fémur

(30) Priority: 26.02.2007 GB 0703691
(43) Date of publication of application: 27.08.2008
(73) Proprietor: BENOIST GIRARD SAS, 14201 Hérouville-Saint-Clair Cédex (FR)
(72) Inventor: Moindreau, Marie, 14000 Caen (FR); Pinot, Loic, 14400 Bayeux (FR); Field, Richard Eddy, Surrey, KT20 7UB (GB); Rushton, Neil, Cambridgeshire, CB2 8AW (GB)
(74) Representative: Bridge-Butler, Alan James

(56) References cited:
- EP-A- 1 348 384
- EP-A- 1 502 550
- WO-A-20/06091704
- DE-B- 1 164 019
- US-A- 6 156 069

## Description

This invention relates to apparatus for preparing a prosthetic stem cavity with inclined sides in the proximal end of a femur when performing proximal epiphyseal replacement technique surgery.

An example of this type of surgery is explained and shown in EP 1138 283 and includes resecting a femur at a position on the proximal side of its neck to locate a prosthetic femoral component which has a tapered insert portion and a proximal head portion. The insert portion is adapted for location in a prepared socket which effectively has inclined sides to provide a tapering opening.

The present invention provides apparatus for preparing such an opening.

WO 2006/091704 A1 shows an in-line milling system for providing a triangular cut in a patient's proximal femur. The apparatus comprises a milling handle, a milling body which is-adapted to fit into a conically reemed femur cavity and a cutting member which are used to mill the triangular cavity. The milling handle and body are manually supported by the surgeon after the reemed femur cavity has been prepared. This construction therefore requires preliminary reeming of the femur cavity and as the apparatus is manually supported inaccuracies can occur.

According to the present invention apparatus for preparing a prosthetic stem cavity with inclined sides in the proximal end of a femur when performing proximal epiphyseal replacement technique surgery comprises drill guide support means which has an upper support part which has a first drill guide for drilling a proximal/distal opening in the proximal end of the bone and a second drill guide for drilling a lateral proximal/distal opening at an angle to said first opening adjacent the proximal end of the bone, a lower part for location on and attachment to the prepared proximal end of a femur and means for locating said drill guide support means at a predetermined angular position about a proximal distal axis on the femur.

The apparatus provides means for accurately drilling the angulated bores in the femur which are subsequently shaped to provide the stem cavity.

If desired the drill guide support means can also include means for transversely guiding a saw to remove proximal bone from the head of the femur before or after drilling.

With this arrangement the said upper part of the drill guide support means can include a saw slot.

In a preferred construction the lower part of the drill guide support means is in the form of a distal attachment collar and the upper part of the drill guide support means can be detachable from the lower part.

The upper part of the drill guide support means can be in the form of a spider with radially projecting arms.

Alternatively, the upper and lower parts of the drill guide support means can be in the form of a monoblock construction.

The drill guide support means can also include means for indicating the bone thickness between the predetermined distal end of the cavity and the outer surface of the femur.

The means for indicating the bone thickness is preferably detachably connected to the drill guide support means and the means for indicating bone thickness may comprise a support member which carries adjustable distance indicating means and in relation to which it can be adjusted to contact the outer surface of the bone.

The adjustable distance indicating means in a preferred construction comprises a sliding pin provided with distance indicating indicea.

The invention can be performed in various ways and two embodiments will now be described by way of example and with reference to the accompanying drawings in which :
Figure 1 is a diagrammatic representation of the proximal end of a femur showing how it is prepared to accept the apparatus according to the invention;
Figure 2 is an exploded isometric view from above showing the various parts of the apparatus according to the invention;
Figure 3 is a view similar to Figure 2 with the parts assembled;
Figure 4 is an isometric view from above of an alternative construction;
Figure 5 is an exploded isometric view of the construction shown in Figure 4;
Figure 6 is a side elevation of the construction shown in Figure 4;
Figure 7 is an exploded side view of the parts as shown in Figure 5;
Figure 8 is a diagrammatic side view showing how some of the parts are assembled onto the femur;
Figure 9 shows all the parts of the construction shown in Figures 4 to 7 assembled on the femur; and,
Figure 10 is a diagrammatic cross-sectional representation of a prosthetic component for use in a prosthetic stem cavity prepared by apparatus according to the invention.

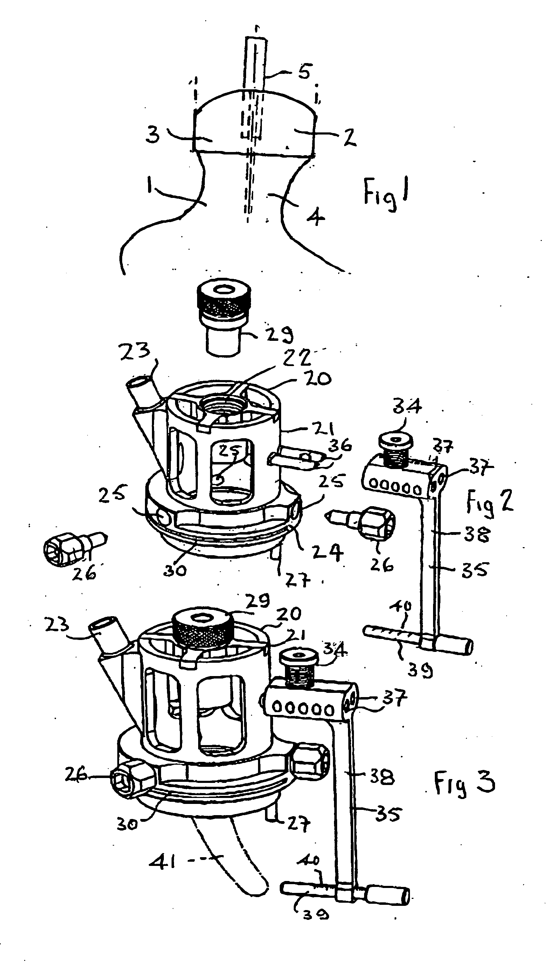

In order to carry out proximal epiphyseal replacement technique surgery on the end of a femur indicated by reference numeral 1 in Figure 1 the femoral head, indicated by reference numeral 2, is first prepared by machining it with a cylindrical cutter (not shown) to provide a cylindrical portion 3. To accurately locate the cylindrical cutter a proximal/distal opening is drilled in the head to receive a guide wire and this opening is subsequently enlarged to receive a guide pin 5 on which the cylindrical cutter is located.

The neck of the femur is indicated by reference numeral 4.

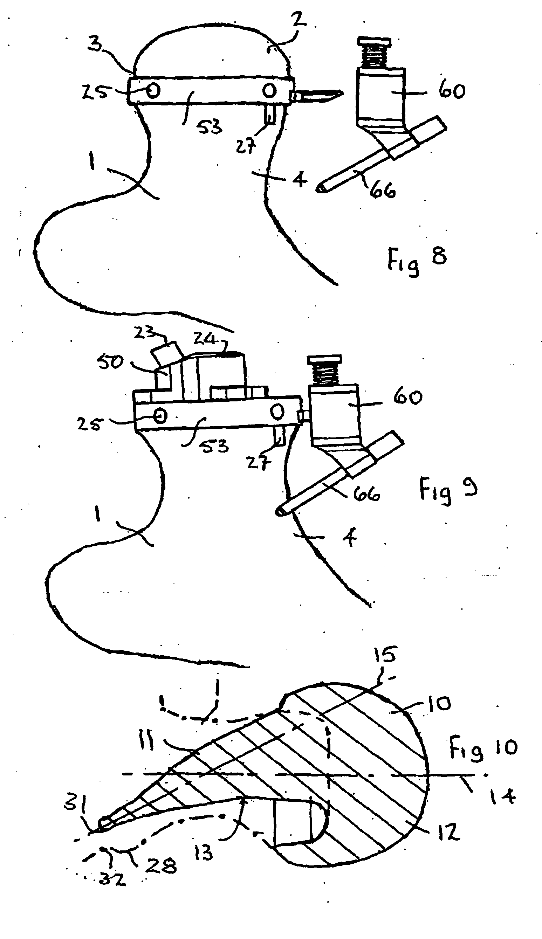

Figure 10 shows diagrammatically how a typical femoral head component which has a tapered insert portion 11 and a head 12 is fitted into a cavity 13 in the proximal end of the femur 1 (indicated by chain lines in Figure 10) according to this type of surgery.

In order to provide the cavity 13 a hole is drilled into the prepared femur along an axis 14 which is substantially co-axial with the proximal/distal axis of the end of the femur. A second opening is also drilled along the line 15 which is at an angle to the line 14 to provide the basis for the tapering socket.

As shown in Figure 2 and 3 a first embodiment of the invention is of a monoblock construction and comprises drill guide support means 20 which have an upper portion 21 in the form of a cylindrical barrel and which has a first drill guide 22 for drilling a proximal/distal opening in the proximal end of the bore along the line 14 as shown in Figure 10. The upper part 21 also has a second drill guide 23 for drilling a lateral/proximal distal opening at an angle to the first opening adjacent the proximal end of the bone and which would produce the opening along the centreline as shown in Figure 10.

A lower part of the drill guide support means 20 is indicated by reference numeral 24 and is the form of a distal attachment collar which is dimensioned to fit accurately over the machined cylindrical portion 3 of the prepared head 2. Three tapped openings 25 are provided into which attachment screws 26 can be threaded to engage the cylindrical portion 3 and locate and attach the drill guide support means to the prepared head 2 of the femur.

Means for locating the drill guide support means 20 at a predetermined angular position about a proximal distal axis on the femur, for example the line 14 of Figure 10, are provided by a downwardly projecting pointer 27 which can be aligned with the calcar, indicated in Figure 10 by reference numeral 28.

The drill guide 22 is provided with a removable centring socket 29 which can be located in the drill guide 22 and which is dimensioned to fit over the guide pin 5 to align the drill guide support means 20 when being fitted. It is then removed along with the guide pin 5.

In order to trim the head 2 a transversely extending saw slot 30 is provided in the lower part 24 and this can be utilized to remove bone from the head of the femur before or after drilling.

The drill guide support means also includes means for indicating the bone thickness between the predetermined distal end of the cavity, indicated by reference numeral 31 of Figure 10, and the outer surface of the bone of the femur, indicated by reference numeral 32. The bone thickness indicator reference numeral 35 is detachably connected to the drill guide support means 20 by two projecting location pins 36 which can engage co-operating openings 37 in a support member 38. The pins are held in position by a spring loaded locking pin 34. The lower end of the support carries adjustable distance indicating means in the form of a sliding pin 39 and which can be adjusted to contact the outer surface of the bone 32 (see Figure 10). The sliding pin 39 is provided with distance indicating indicea 40.

In Figure 3 the support member 38 is shown in place on the drill guide support means 20. With the apparatus in place on the prepared femur head and knowing the dimensions of the various parts it is possible to move the sliding pin 39 until it engages the side of the bone opposite the pre-estimated position of the end of the drilled opening 13. The distance indicating indicea 40 will now provide a reading which will give the thickness of the bone between the end of the opening and the bone wall. Thus, this bone thickness indicator is used to confirm the bone thickness before drilling takes place.

When the openings have been drilled the apparatus is removed and a rasp one size smaller than the definitive size can be used to enlarge the cavity taking care not to enlarge the cavity beyond the medial and lateral limits of the original holes. Such a rasp is shown by broken line 41 in Figure 3.

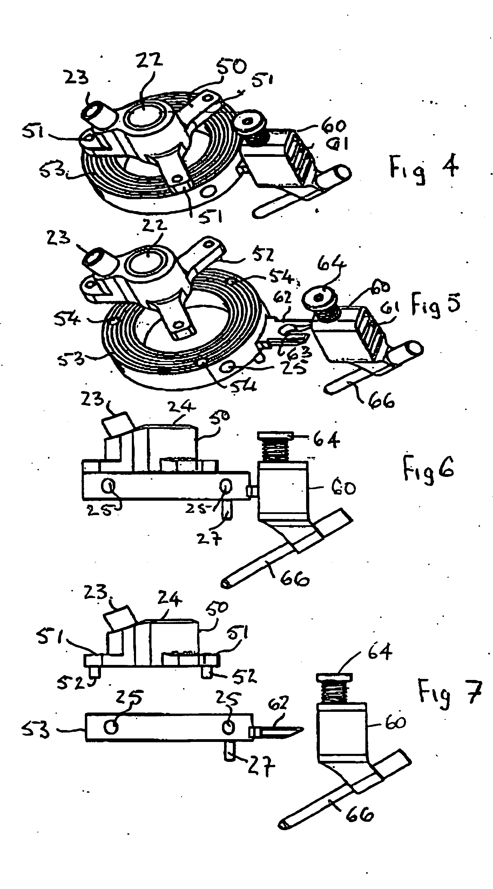

Figures 4 to 9 show an alternative construction embodying the invention and the same reference numerals are used to define similar parts as shown in Figures 2 and 3.

In this construction the drill guides 23 and 22 are carried by drill guide support means 50 in the form of a spider with radially projecting arms 51. Each of the arms has a location pin 52 (best seen in Figure 7) which can engage in the upper surface of a lower part 53 which is in the form of a detachable distal attachment collar. The pins 52 can engage in openings 54 in the attachment collar 53 to locate the spider 50 in position thereon and tapped openings 25 are again provided to receive bolts 26 (not shown in Figures 4 to 9) to locate the distal attachment collar in place on the prepared femur head.

With this construction the means for indicating the bone thickness comprises a support member 60 provided with a series of slots 61 which can appropriately engage a projecting lug 62 on the side of the collar 53 to effectively adjust the proximal/distal length of the support member 60. The lug has an opening 63 to receive a spring loaded locking pin 64 to hold the support member 60 in place thereon. The sliding pin 66 in this construction is arranged at an angle to provide an appropriate engagement with the outer surface of the bone.

The location pin 27 is again provided on the lower part of the support means but no saw slot is included. The upper surface of the attachment collar 53, however, acts as a guide for a saw for removing proximal bone from the head of the femur when required.

In certain circumstances the means for indicating the bone thickness between the predetermined distal end of the cavity and the surface may not be necessary and thus the projecting lug 62 on the side of the collar 53 can be omitted.

Figures 8 and 9 show how this embodiment is used. The head 2 of the femur is prepared as shown in Figure 1 with a cylindrical portion 3 and the distal attachment collar 53 is placed in position using the screws 26. With the collar located in the appropriate position using the guide pin 27 the screws 26 are inserted and the upper surface of the collar is used as a guide to remove proximal bone from the head of the femur. The spider 50 is now placed in position as is the bone thickness indicator 60, the positions checked and drilling can then take place in a similar manner to that described with regard to Figures 2 and 3.

## Claims

1. Apparatus for preparing a prosthetic stem cavity with inclined sides in the proximal end of a femur when performing proximal epiphyseal replacement technique surgery comprising drill guide support means 20 which has an upper part 21 which has a first drill guide 22 for drilling a proximal/distal opening in the proximal end of the bone and a second drill guide 23 for drilling a lateral proximal/distal opening at an angle to said first opening adjacent the proximal end of the bone, a lower part 34 for location on and attachment to the prepared proximal end of a femur and means 27 for locating said drill guide support means 20 at a predetermined angular position about a proximal distal axis on the femur.

2. Apparatus as claimed in claim 1 in which the drill guide support means 20 include means 30 for transversely guiding a saw to remove proximal bone from the head of the femur before or after drilling.

3. Apparatus as claimed in claim2 in which the said upper part 21 includes a saw slot 30.

4. Apparatus as claimed in claim 1, claim 2 or claim 3 in which the lower part 24 of the drill guide support means is in the form of a distal attachment collar 24.

5. Apparatus as claimed in any one of preceding claims 1 to 4 in which said upper part 21 of the drill guide support means 20 is detachable from the said lower part 24.

6. Apparatus as claimed in claim 5 in which said upper part 21 of the drill guide support means 20 is in the form of a spider with radially projecting arms arms

7. Apparatus as claimed in any one of preceding claims 1 to 4 in which the upper 21 and lower 24 parts of the drill guide support means 20 are in the form of a monoblock construction.

8. Apparatus as claimed in any one of the preceding claims in which said drill guide support means 20 includes means 30 for indicating the bone thickness between the predetermined distal end of the cavity and the outer surface of the femur.

9. Apparatus as claimed in claim 8 in which the means 35 for indicating bone thickness is detachably connected to said drill guide support means 30.

10. Apparatus as claimed in claim 8 or claim 9 in which the means 35 for indicating bone thickness comprises a support member which carries adjustable distance indicating means 39 and in relation to which it can be adjusted to contact the outer surface of the bone 32.

11. Apparatus as claimed in claim 10 in which the adjustable distance indicating means 30 comprises a sliding pin 39 provided with distance indicating indicea 40.

## Patentansprüche

1. Vorrichtung zur Herstellung eines für einen prothetischen Schaft bestimmten Hohlraums mit geneigten Seiten im proximalen Ende eines Femur bei der Durchführung einer proximalen Epiphysenersatztechnik-Operation, umfassend ein Bohrführungshaltemittel 20, das aufweist: ein Oberteil 21, welches eine erste Bohrführung 22 zum Bohren einer proximaldistalen Öffnung im proximalen Ende des Knochens und eine zweite Bohrführung 23 zum Bohren einer lateralen proximaldistalen Öffnung unter einem Winkel zu der ersten Öffnung benachbart zum proximalen Ende des Knochens aufweist, ein Unterteil 34 zur Positionierung auf und Befestigung am vorbereiteten proximalen Ende eines Femur, sowie Mittel 27 zum Positionieren des Bohrführungshaltemittels 20 in einer vorbestimmten Winkelposition um eine proximal-distale Achse auf dem Femur.

2. Vorrichtung nach Anspruch 1, bei welcher die Bohrführungshaltemittel 20 Mittel 30 einschließen, um eine Säge quer zu führen, um vor oder nach dem Bohren proximalen Knochen vom Kopf des Femur zu entfernen.

3. Vorrichtung nach Anspruch 2, bei welcher das Oberteil 21 einen Sägeschlitz 30 einschließt.

4. Vorrichtung nach Anspruch 1, Anspruch 2 oder Anspruch 3, bei welcher das Unterteil 24 des Bohrführungshaltemittels in Form eines distalen Befestigungskragens 24 vorliegt.

5. Vorrichtung nach einem der vorangehenden Ansprüche 1 bis 4, bei welcher das Oberteil 21 des Bohrführungshaltemittels 20 vom Unterteil 24 abnehmbar ist.

6. Vorrichtung nach Anspruch 5, bei welcher das Oberteil 21 des Bohrführungshaltemittels 20 in Form einer Spinne mit radial überstehenden Armen vorliegt.

7. Vorrichtung nach einem der vorangehenden Ansprüche 1 bis 4, bei welcher das Oberteil 21 und das Unterteil 24 des Bohrführungshaltemittels 20 in Form einer Monoblockkonstruktion vorliegen.

8. Vorrichtung nach einem der vorangehenden Ansprüche, bei welcher das Bohrführungshaltemittel 20 ein Mittel 30 zum Anzeigen der Knochendicke zwischen dem vorbestimmten distalen Ende des Hohlraums und der äußeren Oberfläche des Femur einschließt.

9. Vorrichtung nach Anspruch 8, bei welcher das Mittel 35 zum Anzeigen der Knochendicke abnehmbar mit dem Bohrführungshaltemittel 30 verbunden ist.

10. Vorrichtung nach Anspruch 8 oder Anspruch 9, bei welcher das Mittel 35 zum Anzeigen der Knochendicke ein Halteelement umfasst, das ein verstellbares Entfernungsanzeigemittel 39 trägt, und in Bezug zu dem es eingestellt werden kann, um mit der äußeren Oberfläche des Knochens 32 in Kontakt zu treten.

11. Vorrichtung nach Anspruch 10, bei welcher das verstellbare Entfernungsanzeigemittel 30 einen Schiebestift 39 umfasst, der mit Entfernungsanzeigemarkierungen 40 versehen ist.

## Revendications

1. Appareil pour ménager une cavité à côtés inclinés pour tige de prothèse dans l'extrémité proximale d'un fémur à l'occasion d'une opération chirurgicale par une technique de remplacement de l'épiphyse proximale, comprenant un moyen de support (20) de guide-mèches ayant une partie supérieure (21) munie d'un premier guide-mèche (22) pour forer une ouverture proximale/distale dans l'extrémité proximale de l'os et d'un second guide-mèche (23) pour forer une ouverture latérale proximale/distale de manière oblique par rapport à ladite première ouverture au voisinage immédiat de l'extrémité proximale de l'os, une partie inférieure (34) à placer sur et à fixer à l'extrémité proximale préparée d'un fémur et un moyen pour placer ledit moyen de support (20) de guide-mèches dans une position angulaire prédéterminée autour d'un axe proximo-distal sur le fémur.

2. Appareil selon la revendication 1, dans lequel le moyen de support (20) de guide-mèches comprend un moyen (30) pour guider transversalement une scie afin de retirer de l'os proximal de la tête du fémur avant et après le forage.

3. Appareil selon la revendication 2, dans lequel ladite partie supérieure (21) comporte une encoche (30) pour scie.

4. Appareil selon la revendication 1, la revendication 2 ou la revendication 3, dans lequel la partie inférieure (24) du moyen de support de guide-mèches présente la forme d'un collier de fixation distal (24).

5. Appareil selon l'une quelconque des revendications précédentes 1 à 4, dans lequel ladite partie supérieure (21) du moyen de support (20) de guide-mèches est détachable de ladite partie inférieure (24).

6. Appareil selon la revendication 5, dans lequel ladite partie supérieure (21) du moyen de support (20) de guide-mèches présente la forme d'un croisillon à branches déployées radialement.

7. Appareil selon l'une quelconque des revendications précédentes 1 à 4, dans lequel les parties supérieure (21) et inférieure (24) du moyen de support (20) de guide-mèches présentent la forme d'une construction monobloc.

8. Appareil selon l'une quelconque des revendications précédentes, dans lequel ledit moyen de support (20) de guide-mèches comporte un moyen (30) pour indiquer l'épaisseur de l'os entre l'extrémité distale prédéterminée de la cavité et la surface extérieure du fémur.

9. Appareil selon la revendication 8, dans lequel le moyen (35) pour indiquer l'épaisseur de l'os est monté de manière amovible sur ledit moyen de support (30) de guide-mèches.

10. Appareil selon la revendication 8 ou la revendication 9, dans lequel le moyen (35) pour indiquer l'épaisseur de l'os comporte un élément de support qui porte un moyen réglable (39) d'indication de distance et par rapport auquel il peut être réglé pour venir au contact de la surface extérieure de l'os (32).

11. Appareil selon la revendication 10, dans lequel le moyen réglable (30) d'indication de distance comprend une tige coulissante (39) munie de repères (40) d'indication de distance.
